(19) Europäisches Patentamt
European Patent Office
Office européen des brevets

(11) **EP 4 072 107 A1**

(12) **EUROPEAN PATENT APPLICATION**

(43) Date of publication:
**12.10.2022 Bulletin 2022/41**

(21) Application number: **22166727.2**

(22) Date of filing: **05.04.2022**

(51) International Patent Classification (IPC):
**H04L 67/12** *(2022.01)*   **H04W 4/029** *(2018.01)*
**H04W 4/90** *(2018.01)*   **G16H 50/80** *(2018.01)*

(52) Cooperative Patent Classification (CPC):
**H04W 4/90; G16H 50/30; G16H 50/80; H04L 67/12; H04W 4/029**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.04.2021 US 202163171559 P**

(71) Applicant: **The Boeing Company**
**Chicago, IL 60606-1596 (US)**

(72) Inventors:
- **Austin, Thomas Robert**
  **Chicago, 60606-1596 (US)**
- **Mereness, Rebecca Ann**
  **Chicago, 60606-1596 (US)**
- **Pepper, Graig Anthony**
  **Chicago, 60606-1596 (US)**
- **Svenson, Dale V.**
  **Chicago, 60606-1596 (US)**

(74) Representative: **Arnold & Siedsma**
**Bezuidenhoutseweg 57**
**2594 AC The Hague (NL)**

(54) **SYSTEMS AND METHOD FOR REDUCING HEALTH THREATS WHEN TRAVELING**

(57)    A system and method include one or more processors configured to determine a risk index associated with a risk of exposure to a health threat for each node within a chain of nodes during a stage of a journey. The risk index for each node is based on occupancy characteristics of the node, a set of one or more control measures in effect to reduce the risk of exposure in the node, and a health threat prevalence in a jurisdiction associated with the node. The one or more processors are further configured to aggregate the risk indices for the nodes to determine a risk value associated with the stage of the journey, and to generate a control signal to notify a user of the risk value associated with the stage of the journey.

FIG. 1

EP 4 072 107 A1

**Description**

FIELD OF THE DISCLOSURE

Examples of the present disclosure generally relate to transportation systems, such as commercial aircrafts and other modes of transportation, as well as and airports and other transportation hubs, and simulating the effectiveness of control measures at reducing health threat risks for people present within the transportation systems.

BACKGROUND OF THE DISCLOSURE

The perceived risk of health threats within public transportation systems can affect the number of travelers using conventional modes of transportation, such as taking commercial flights. An example health threat is exposure to pathogens or contagions associated with infectious diseases. In general, and particularly when there is an infectious disease outbreak, public transportation systems, including airports and commercial airlines, may implement various control measures designed to reduce the risk of pathogen exposure and, therefore, increase the confidence of travelers. Example control measures can include limiting occupancy, providing designated place markers for travelers waiting in lines or queues, requiring occupants to wear masks and/or be vaccinated, encouraging occupants to practice social distancing, requiring spacing between passengers (e.g., keeping middle seats of rows unoccupied), and the like. Different control measures have different levels of effectiveness at reducing the risk of pathogen exposure, different costs to implement (e.g., loss of revenue by accommodating fewer passengers), and different levels of acceptance by the travelers and employees that are affected by the control measures.

It would not be economically or operationally feasible for public transportation companies to implement all available control measures for an extended period of time, such as limiting commercial flights to less than half of the capacity in perpetuity. It is difficult to determine which specific control measures, implemented alone or in combination, would be more effective at reducing the risk of pathogen exposure without substantially disrupting the travelers or reducing revenues, relative to other specific control measures. For example, there are a large number of variables that can affect the risk of pathogen exposure throughout a journey of a traveler, which can include multiple different modes of transportation. A traveler can be exposed to health risks during interactions with others and via encounters with hazardous surfaces within each vehicle and within the transportation facilities (e.g., airports). Known modeling techniques are limited in scope, such as evaluating the risk at only a single location and/or in a single vehicle. The known modeling techniques fail to account for various factors that are relevant to assessing risk for an entire traveler's journey, so have limited accuracy and utility.

SUMMARY OF THE DISCLOSURE

A need exists for a system and a method for modeling the risk of a health threat for travelers that has the ability to represent the complete end-to-end journeys of travelers, including multiple modes of transportation. Further, a need exists for identifying cost-effective and high-value control measures that would reduce the risk of health threats for travelers while having a low adverse impact on the transportation systems.

With those needs in mind, certain examples of the present disclosure provide a system that includes one or more processors configured to determine a risk index associated with a risk of exposure to a health threat for each node within a chain of nodes during a stage of a journey. The risk index for each node is based on occupancy characteristics of the node, a set of one or more control measures in effect to reduce the risk of exposure in the node, and a health threat prevalence in a jurisdiction associated with the node. The one or more processors are further configured to aggregate the risk indices for the nodes in the chain to determine a risk value associated with the stage of the journey, and to generate a control signal to notify a user of the risk value associated with the stage of the journey.

Certain examples of the present disclosure provide a method that includes determining, via a computer-implemented model, a risk index associated with a risk of exposure to a health threat for each node within a chain of nodes during a stage of a journey. The risk index for each node is determined based on occupancy characteristics of the node, a set of one or more control measures in effect to reduce the risk of exposure in the node, and a health threat prevalence in a jurisdiction associated with the node. The method includes aggregating the risk indices for the nodes to determine a risk value associated with the stage of the journey, and generating a control signal to notify a user of the risk value associated with the stage of the journey.

Certain examples of the present disclosure provide a system that includes a display device and one or more processors communicatively connected to the display device. The one or more processors are configured to determine a risk index associated with a risk of exposure to a health threat for each node within a chain of nodes during a journey. The risk index for each node is based on occupancy characteristics of the node, a set of one or more control measures in effect to reduce the risk of exposure in the node, and a health threat prevalence in a jurisdiction associated with the node. The one or more processors are further configured to aggregate the risk indices for the nodes to determine a risk value associated with the journey and the set of one or more control measures, and to determine an adverse impact associated with implementing the set of one or more control measures. The one or more processors are configured to calculate a net value for the set of control measures based on the risk value and the adverse impact, and to display the net value on the display device.

BRIEF DESCRIPTION OF THE DRAWINGS

Figure 1 is a block diagram of a risk assessment system.

Figure 2 is a schematic diagram that illustrates deterministic compartmental elements of a risk model of the risk assessment system.

Figure 3 is a diagram showing four different node maps.

Figure 4 illustrates an air conditioning zone within an airport.

Figure 5 illustrates an enlarged view of a checked bag queue node shown in Figure 4.

Figure 6 illustrates a portion of the risk assessment system including graphical analytics.

Figure 7 is a flow chart of a method for travel risk assessment.

DETAILED DESCRIPTION OF THE DISCLOSURE

The foregoing summary, as well as the following detailed description of certain examples will be better understood when read in conjunction with the appended drawings. As used herein, an element or step recited in the singular and preceded by the word "a" or "an" should be understood as not necessarily excluding the plural of the elements or steps. Further, references to "one example" are not intended to be interpreted as excluding the existence of additional examples that also incorporate the recited features. Moreover, unless explicitly stated to the contrary, examples "comprising" or "having" an element or a plurality of elements having a particular condition can include additional elements not having that condition.

Certain examples of the present disclosure provide a system and method for assessing and reducing health threats when traveling. The system may be referred to as a travel risk assessment system. For example, the system and method may simulate the risk of health threats along journeys and then implement measures, based on the simulated risk assessment, designed to reduce the risk of health threats encountered by travelers on the journeys. The health threats described herein may include various unsafe conditions, such as exposures to pathogens or contagions that can cause infectious disease or illness, exposures to toxic gases, slipping or tripping hazards, hazardous conditions for disabled people, and the like. Although examples described herein refer to risk of exposure to pathogens, the health threats that are evaluated by the system and method described herein are not limited to risk of pathogen exposure. The journeys described herein may encompass multiple stages or legs of a trip from a starting location, such as a traveler's house, to a destination location, such as a hotel in another state or country from the traveler's residence. For example, a journey may include ground-based travel from the residence to a departure airport, presence within the departure airport prior to a flight, the flight to an arrival airport, presence within the arrival airport, and then ground-based travel to a hotel or other destination location.

The system and method may simulate the risk of health threats using a computer-implemented model, referred to herein as a risk model. The risk model, according to a non-limiting example, may be an adaptive, multi-disciplinary, software-based, risk management tool designed to provide risk-informed decision support and restore safety, confidence and convenience in commercial travel, including aviation. The risk model may model the relative effectiveness of layered control measures throughout end-to-end journeys in global transportation systems. The control measures represent rules, actions, and/or restrictions that may be enacted and/or performed in an attempt to reduce the risk of exposure to health threats. With respect to infectious disease-causing pathogens, the control measures may be designed to thwart the spread of respiratory plumes from infected hosts as well as surface-born exposures. The control measures may include frequent sanitizing, social distancing, mask-wearing, air flow management and filtering, touchless technologies, vaccination, secure credentialing, reduced capacity restrictions in buildings and public vehicles, restrictions against eating or drinking, on-site health screening, and the like. Optionally, the risk model may employ airborne and surface-borne disease exposure sub-models in a deterministic modular approach.

For each journey of a traveler from a starting location to a destination location, the risk model may use nodal progression to evaluate the risk of a health threat at each node in a chain of nodes potentially occupied by the traveler over time. The nodes in this context may refer to specific places or areas. Optionally, some nodes may be characterized based on people that occupy the node and the activity of the people that occupy that node. The system described herein may segment interior spaces of buildings and vehicles into multiple discrete nodes. An airport can be segmented into dozens or even hundreds of nodes, such as nodes within check-in lines or queues, security screenings, waiting areas, lavatories, restaurants, and the like. For a given travel journey, the system may determine a chain of the nodes which represents a path that the traveler is expected to take through the various environments, such as through an airport. The expected path of the traveler may be based on trip characteristics, such as an entrance location through which the travelers is expected to enter the airport, whether the traveler is flying domestic or international, the length of time that the traveler will be in the airport before a flight, the gate from which the traveler will board the flight, and the like.

According to an example, the system may implement the risk model to determine a risk of exposure to a health threat for the traveler at each node in the chain associated with the traveler. For example, the system may determine a risk of exposure to a health threat while the traveler waits in a line or queue for security screening, and may determine another risk of exposure to a health threat while the traveler is at a restaurant in the airport. The risk of exposure to a health threat determined for a given node may be characterized as a risk index. The risk index may be a quantitative value assigned by the risk model as an output, as described herein. In an example, the risk indices for the nodes may be relative risk indices, as opposed to absolute risk indices. For example, the relative risk index for a given node may represent whether the particular set of conditions at the node has a higher or lower risk of a health threat to the traveler than another set of conditions at the node, such as a different set of control measures in place, or than the risk of the health threat at a benchmark location outside of the stage of the journey, such as at a restaurant, a sporting event. or the like.

Upon determining the risk indices for the nodes in the chain of nodes associated with the predicted movement of the traveler, the system may aggregate the risk indices for the nodes in the chain. The risk indices may be aggregated by summation or by other statistical calculations, such as mean, weighted mean, or the like. The system may aggregate the risk indices to determine a risk value associated with the journey. The risk value may be a relative value that is based on a comparison to other risk values calculated for the same journey under different sets of conditions and/or calculated for different journeys or benchmark locations. Some examples described herein refer to relative risk indices, but the risk assessment system is not limited to relative risk indices, and may include absolute risk indices.

In an example, the system may compartmentalize the journey of a traveler into multiple different stages. For example, a first stage may represent movement of the traveler to a departure airport, a second stage may represent activity or presence of the traveler within the departure airport, a third stage may represent one or more flights of the traveler to an arrival airport, a fourth stage may represent activity or presence of the traveler within the arrival airport, and a fifth stage may represent movement of the traveler from the arrival airport. The movement of the first and fifth stages may be via ground-based or water-based modes of transportation, such as car, bus, train, or water vessel. The system described herein may perform nodal-based progression to determine the relative risk indices for each discrete stage, and calculate a respective risk value for each stage by aggregating the relative risk indices associated with that stage. Optionally, the system may aggregate the relative risk indices for all nodes throughout the entire journey to determine an overall risk value for the journey.

The system may generate a control signal to notify a user (e.g., a human operator) about the risk value(s) associated with the journey, such as the risk values for the stages of the journey. The control signal may cause a display device to display the risk value(s) on a screen of the display device to be viewable by the user. Optionally, the control signal may provide an audible, speech-based notification of the risk value(s) to the user via the use of audio speakers, such as headphones. The risk values may be presented to the user to recommend cost-effective, best-value solutions for decision makers (e.g., transportation company managers, politicians, and other policy makers). Optionally, the risk values may be presented to a traveler to inform the traveler about health risks involved in a perspective stage of a journey and enable the traveler to modify a stage to reduce the health risk.

The system may enable identification and prioritization of a set of one or more specific risk-reduction control measures that is expected to provide greater net value than if other sets of risk-reduction control measures are in place for the journey. The net value may represent an amount of risk reduction or health threat avoidance relative to potential adverse impacts that the set of control measures would have on the commercial transportation ecosystem. The amount of risk reduction may be characterized by the risk values determined using the risk model and nodal progression along the journey. The system according to an example may be augmented by integration of multiple parameters that complement risk management, including economic and operational cost impact, expected travel delays, perceived experiences of hassle, and psychological impacts of fear and confidence associated with the control measures. The parameters may also be classified according to the perspectives of different stakeholders, including travelers, government agencies, airport management, airline management, airline manufacturers, and the travel industry.

For example, the system may associate each prospective control measure with a corresponding economic and operational impact assessment. The economic and operational impact assessment may be determined using quantitative and/or qualitative analysis, and can provide information about the potential cost in terms of tangible costs and intangible costs. The tangible costs may include the cost to implement and regulate the control measure, the cost of lost revenue as a result of the control measure, and the like. The intangible costs may include the hassle of travelers and other stakeholders to comply with the control measure, expected delays attributable to the control measure, fear invoked in travelers due to the control measure, and/or general negative sentiment directed to the transportation systems as a result of implementing the control measure. In an example, the economic and operational impact assessments may be determined at least in part by surveying experts, such as experts in the fields of transportation systems, health policy, and/or the like, to rate various control measures based on several factors to determine a qualitative economic and operational impact assessment for each control measure. Optionally, stakeholders that would be affected by the control measures may be surveyed as well, such as travelers, airline management, airport management, and the like.

By factoring both the amount of risk reduction and the potential adverse impacts for implementing a given set of control measures, the system disclosed herein may enable informed decision-making. For example, the system may explicitly recommend a set of control measures to implement that has greater net value than other potential sets of control measures. Alternatively, the system may provide information to a user for the user to make an informed decision about which set of control measures should be implemented to provide a greater net value than other sets of control measures. The recommended set of control measures may not necessarily be the set that provides the absolute greatest risk reduction possible, and may not be the set that provides the absolute least adverse impact on the commercial transportation ecosystem. Rather, the recommended set of control measures may be a set that is predicted to provide greater risk reduction relative to the adverse impact (e.g., net value) when compared to other tested sets of control measures. The system may generate a control signal to notify a user of the recommended set of control measures, such as by displaying a text-based message on a display device. Optionally, the system may be configured to automatically save the risk values for a given journey and/or the recommended set of control measures on a local data storage device (e.g., memory) and/or may automatically communicate such information to a remote device, such as a server. Optionally, the system may generate a prompt that is displayed on the display device to the user, where the prompt asks the user whether the user would like to communicate the risk values and/or recommended set of control measures.

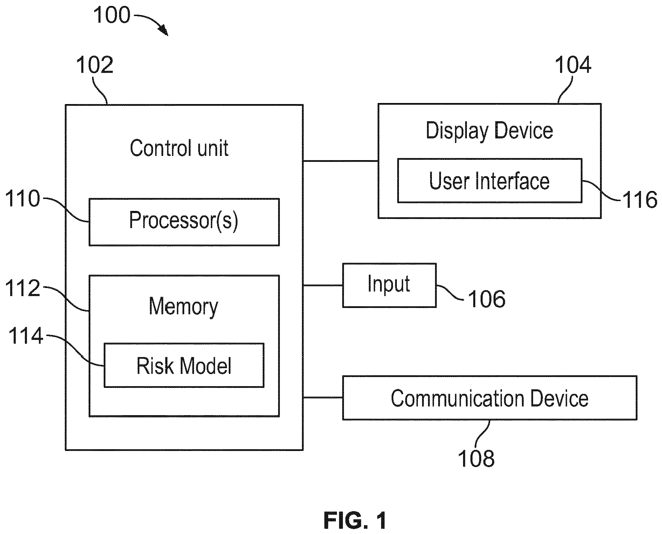

Figure 1 is a block diagram of a risk assessment system 100 according to an example. The risk assessment system 100 in the illustrated example includes a control unit 102 (or controller), a display device 104, an input device 106, and a communication device 108. The control unit 102 is communicatively connected to each of the display device 104, the input device 106, and the communication device 108. The control unit 102 represents hardware circuitry that includes and/or is connected with one or more processors 110 (e.g., one or more microprocessors, integrated circuits, microcontrollers, field programmable gate arrays, etc.). The control unit 102 includes and/or is connected with at least one tangible and non-transitory computer-readable storage medium (e.g., memory) 112. For example, the memory 112 may store programmed instructions (e.g., software) that is executed by the one or more processors 110 to perform the operations of the control unit 102 described herein. Although the processors 110 and the memory 112 are illustrated as two different components in Figure 1, the components optionally may be integrated into a single processing component with data storage functionality. The control unit 102 may be a portion of a computing device.

In an example, the memory 112 may store a risk model 114 which performs risk-based analyses of health threats when traveling, as described above. The risk model 114 may be an adaptive, multi-disciplinary, software-based, risk management tool designed to provide risk-informed decision support and restore safety, confidence and convenience in commercial travel, including aviation. The one or more processors may utilize the risk model 114 in the memory 112 to provide information to users regarding which set of one or more control measures would provide more net value than other available sets of control measures during modeled journeys. In an alternative example, the risk model 114 may be hard-wired into the logic of the one or more processors 110 instead of stored in the memory 112.

The display device 104 may be a monitor or other hardware that has a display screen configured to display a user interface 116, such as a graphical user interface (GUI). The display device 104 optionally may be integrated into a common computing device with the control unit 102, or may be a discrete from the control unit 102 but communicatively connected via an electrically conductive pathway or a wireless pathway. In an example, the control unit 102 displays information generated by the risk model 114 on the user interface 116 of the display device 104 for presentation to a user (e.g., human operator) viewing the display device 104. The user interface 116 may be updated in real-time by selecting different parameters, such as different sets of control measures to implement. For example, a user may toggle between different scenarios to compare and contrast the modeled relative risk assessments, for planning purposes. The display device 104 optionally may be a component of a mobile device, such as a smart phone, smart glasses or a smart watch.

The input device 106 may include a keyboard, a touchpad, a touchscreen (e.g., with virtual keys), a button, a mouse, a joystick, or the like. The input device 106 is configured to receive user touch inputs from a user and generate signals indicative of the user touch inputs that are communicated to the control unit 102 via wired or wireless communication pathways.

The communication device 108 is configured to communicate messages with remote devices. The control unit 102 may generate control signals that cause the communication device 108 to transmit messages to a remote device, such as a server in a remote data facility. The communication device 108 may also be configured to receive messages and forward the messages to the control unit 102 for processing and analysis of the messages. The communication device 108 can include a transceiver that both sends and receives messages, or may have a receiver and a discrete transmitter that separately perform the receive and send functions. The communication device 108 may also include associated circuitry and other hardware, such as one or more antennas, amplifiers, power supply devices, or the like.

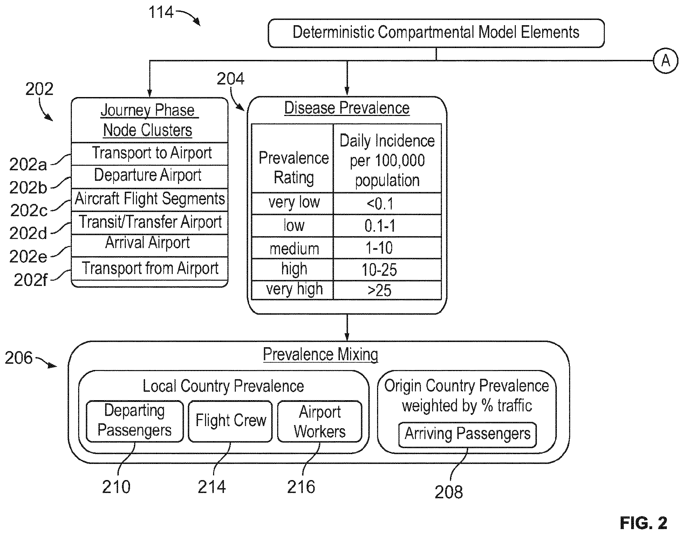

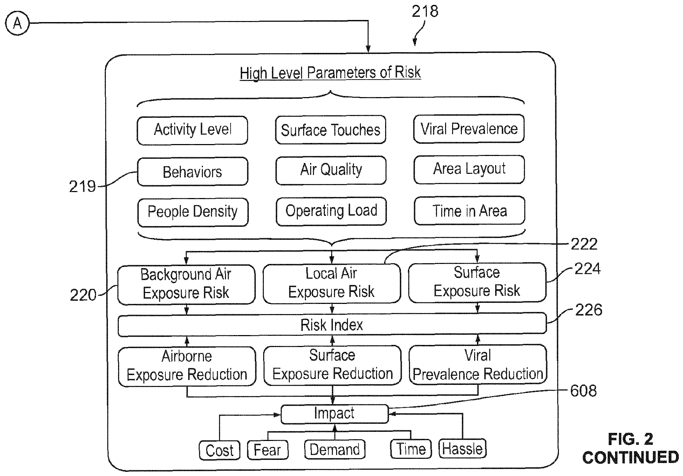

Figure 2 is a schematic diagram that illustrates deterministic compartmental elements of the risk model 114 according to an example. In an example, the model identifies a multitude of nodes, and groups the nodes into discrete stages 202 of a journey. The stages 202 in Figure 2 are classified as transport to departure airport 202a, departure airport 202b, aircraft flight segments 202c, transit/transfer airport 202d, arrival airport 202e, and transport from arrival airport 202f. In an example, the risk model 114 determines a relative risk index, associated with a risk of exposure to a health threat, for each node in the respective stage 202.

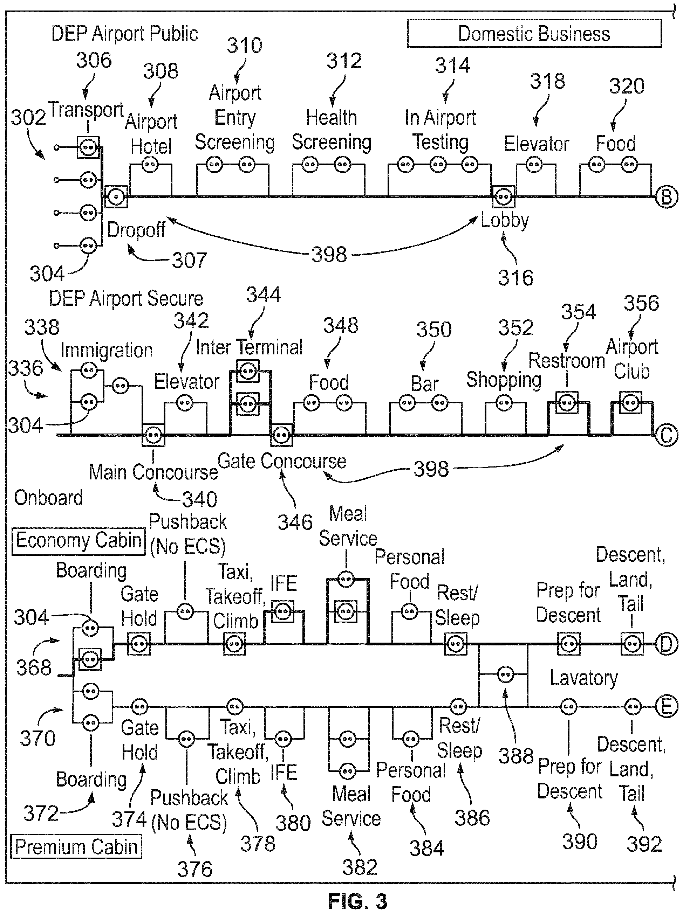

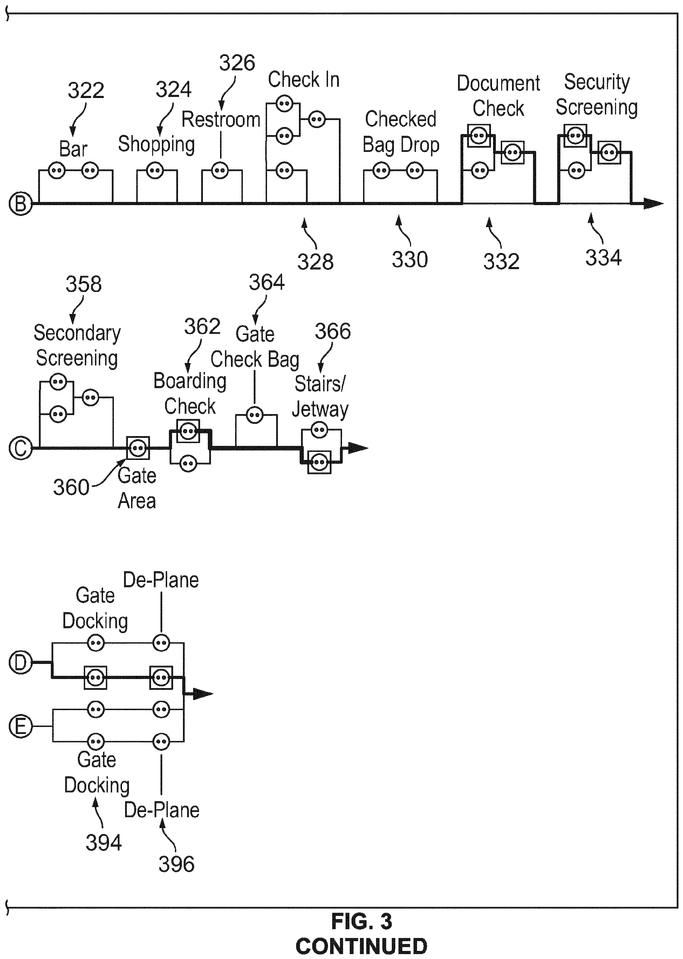

Figure 3 is a diagram showing four different node maps 302, 336, 368, 370 according to an example. For example, the risk model 114 may be designed to incorporate or access predetermined node maps for different facilities and vehicles, such as airports and commercial aircraft. The node maps may be designed specific to a particular facility (e.g., John F. Kennedy Airport) or type of vehicle, or may be generic. Figure 3 illustrates a first node map 302 that represents a public section of a departure ("DEP") airport for a journey. The first node map 302 includes various nodes 304 associated with different locations, people, and/or activity within the public section of the airport. The illustrated nodes 304 in Figure 3 include type of transport to the public section of the airport 306, drop-off at the airport 307, airport hotel 308, airport entry screening 310, health screening 312, in-airport testing 314, lobby 316, elevator 318, food 320, bar 322, shopping 324, restroom 326, flight check-in counter 328, checked baggage drop 330, document check 332, and security screening 334.

A second node map 336 represents a private section of the departure airport, such as after passing through security. The nodes 304 in the second node map 336 in Figure 3 include immigration 338, main concourse 340, elevator 342, international terminal 344, gate concourse 346, food 348, bar 350, shopping 352, restroom 354, airport club 356, secondary screening 358, gate area 360, boarding queue 362, gate check bag 364, and jetway 366. The specific nodes shown in Figure 3 are for example only, and indicate how the airport can be categorized or segmented into an array of dozens of nodes. Both the first and second node maps 302, 336 represent portions of the departure airport stage 202b of the journey shown in Figure 2.

The third node map 368 and the fourth node map 370 in Figure 3 represent the third stage 202c of the journey in which the traveler is onboard a commercial aircraft for a flight. The third and fourth node maps 368, 370 may be alternative options, such that a traveler would only experience one of the two node map 367, 370 during the stage. The third node map 368 refers to the traveler having a ticket in an economy cabin, and the fourth node map 370 refers to the traveler having a ticket in a premium cabin. The nodes 304 in the third node map 368 generally match the nodes 304 in the fourth node map 370, and include boarding 372, gate hold 374, pushback 376, taxi and takeoff 378, IFE 380, meal service 382, personal food 384, rest/sleep 386, lavatory 388, preparation for descent 390, landing and taxi 392, gate docking 394, and de-planing or exiting the aircraft 396. It is noted that nodes 304 may be classified based on activity or location. Multiple nodes 304 can be defined at a common location of the traveler. For example, the traveler may be seated in an assigned seat during the occurrence of most of the nodes 304 in the node maps 368, 370. The risk model 114 may have node maps for the other stages of the journey (e.g., stages 202a, 202d, 202e, and 202f) as well.

The risk model 114 may predict a progression of a specific traveler through the node maps to generate a chain of nodes 398 that the traveler is predicted to encounter. The progression may be predicted based on journey characteristics that are input into the risk model 114. Example journey characteristics can include a time at which the traveler is expected to arrive at the airport, a scheduled time of departure of the flight out of the airport, whether the flight out of the airport is domestic or international, whether the traveler is alone or is traveling with others, such as children, how the traveler is expected to arrive at the airport (e.g., what mode of transportation), whether the traveler is bringing a bag to check, current average wait times within the airport for check-in, security, and health screening, and the like. At least some of this information may be provided by ticketing data. The chain of nodes 398 may be based on historical data of monitored movements of other travelers through the same or similar environments.

In the illustrated example, the nodes 304 that are selected for the chain of nodes 398 in each node map, according to the risk model 114, are indicated by boxes that surround the circular node markers. The nodes that are not boxed represent nodes that are not included within the nodal chain 398. In the first node map 302, the chain 398 includes a first transport node 306, the drop-off node 307, the lobby node 316, two document check nodes 332, and two security screening nodes 334. The first transport node 306 optionally may represent a private car, such as the traveler's own car or a ride-sharing car. The traveler may use online check-in, enabling the traveler to skip the check-in nodes 328. In general, reducing the number of nodes 304 encountered by the traveler may reduce the risk of exposure to a health threat. On the secure side of the airport, the chain of nodes 398 in the predicted progression may include the main concourse 340, international terminal 344, gate concourse 346, restroom 354, airport club 356, gate area 360, a priority boarding queue 362, and a priority jetway 366. Other nodal chains may be determined by the risk model 114 for the other stages of the journey.

Referring now back to Figure 2, the risk model 114 may incorporate jurisdictional prevalence data associated with the health threat (e.g., health threat prevalence). When the health threat is an outbreak of an infectious disease, the jurisdictional prevalence may be disease prevalence data 204, indicating how widespread the disease is in the local community of the jurisdiction at the present time. The prevalence data may be obtained from public or private sources that aggregate the data, such as government health agencies. The risk model 114 may also obtain and use vaccination prevalence data to determine the risk indices. For example, the risk indices may decrease as the percentage of vaccinated individuals in the community increases and/or the effectiveness of vaccination is determined to be high. The risk model 114 may be updated based on new data received that reflects jurisdictional health threat prevalence data and vaccination prevalence data.

In one or more examples, the model 114 employs a deterministic modular approach with group structured mixing. For example, the model 114 may account for various different groups 206 of people that may be present in the environment where there is a potential risk of a health threat. The groups 206 may include arriving passengers 208, departing passengers 210, flight crew 214, non-flight crew airport workers 216, and/or the like. Local health threat prevalence data may be employed to accurately estimate disease exposure risk by populations to and from specific jurisdictions, such as states and countries. For example, departing passengers 210, and airport workers 216 may be characterized by the local prevalence data associated with the location of the airport. Flight crews 214 may be characterized by the local prevalence data associated with the home base of the airline. For arriving passengers 208 at the airport, the risk model 114 may calculate a weighted sum of local country or state prevalence data representing the sources of the arriving passengers 208.

In an example, the risk model 114 performs a risk assessment for each node 304 in the chain 398 described in Figure 3. The risk assessment may factor various (high level) parameters of risk 218 for each node. The parameters of risk 218 may include activity level, behaviors, people density, surface touches, air quality, operating load, health threat prevalence (e.g., viral prevalence), area layout, time in area, and/or the like. Some of the parameters, such as activity level, behaviors, people density, operating load, area layout, and time in area are also referred to herein as occupancy characteristics of a node. Air quality is also referred to herein as an air flow characteristic, along with a flow rate of air, air filtering processes, air replacement rate, and the like.

The risk model 114 may model airborne and/or surface borne threat exposures. The risk model 114 may include a background environmental air exposure risk sub-model 220, a local air exposure risk sub-model 222, and a surface borne exposure risk sub-model 224. The sub-models may be algorithms and/or calculations performed by the model 114. The background air exposure risk sub-model 220 may model aerosol-transported pathogens throughout a large air volume shared with persons in a common air handling zone servicing multiple nodes. The local air exposure risk sub-model 222 may model droplet and aerosol-transported pathogens between close proximity persons in the same node within a small cylindrical zone. The surface borne exposure sub-model 224 may model transmission via surface-to-body (e.g., hand), and then to mucous membranes. In an example, the risk model 114 may calculate airborne disease exposure risk driven by droplet and aerosol mechanisms and disease exposure risk on surfaces based on user-specified weightings.

The control measures may be factored into the sub-models 220, 222, 224, such that performance characteristics of control measures are integrated into the airborne and surface borne disease transmission risk calculations. The control measures in place may be inputs into the risk model 114. At least some of the control measures may be selected by a user via the input device 106. The risk model 114 may include an extensive list of potential control measures that can be implemented, and the model can evaluate different selected sets of one or more of the control measures. The control measures that can be selected may include, without limitation, offsite testing, in-airport testing, staff testing, disinfection of surfaces, body temperature scanning, health and travel history screening, occupancy restrictions, dual boarding, touchless access and payments, physical barriers, suspension of amenities, social distancing, mask-wearing, personal sanitizing at stations, and the like.

The behavior parameter 219 may refer to human behavior characterized by orientation, activities, and spacing of people (e.g., even, queued, or clumped). Behavior may also refer to compliance with individual control measures, such as a percentage of people that comply with a control measure. The behavioral compliance used by the risk model 114 may be based on a specific control measure, stakeholder group (e.g., passengers, flight crew, airport workers, etc.), and journey location or jurisdiction. For example, travelers in or from one country may be more likely to comply with a given control measure than travelers in or from another country. Lack of compliance with a control measure designed to reduce the risk of exposure to a health threat may increase the risk of exposure relative to a greater level of compliance with the control measure. The risk model 114 may incorporate these relationships into the risk assessment calculations.

The risk model 114 is configured to generate a risk index 226 based on the sub-models 220, 222, 224, which factors the set of control measures and the prevalence data 204. The risk index 226 may be determined on a node-by-node basis. After determining the risk index 226 for each node 304 in the chain 398 of a stage of the journey, the one or more processors 110 (or the risk model 114) may aggregate the risk indices 226 of the nodes 304 in the chain 398 to calculate a risk value for the stage of the journey. For example, the risk value may be a product of the cumulative risk indices calculated across multiple successive nodes in the nodal progression (e.g., chain) throughout the stage of the journey. The risk model 114 may perform the operations described above for each of the stages 202 of the journey.

In an example, risk indices of the nodes are relative risk indices that are relative to reference risk indices. For example, the risk model 114 may utilize reference or benchmark risk indices that are determined for benchmark locations outside the stage of the journey. In the described example, the benchmark locations may be outside of the air travel system. The benchmark locations may include restaurants, offices, sporting events, places of worship, public spaces, conference rooms, and/or the like. The relative risk index for each node of a stage may represent the risk of exposure at the respective node compared to the risk of exposure at one or more of the benchmark locations. For example, the relative risk index for the node associated with the security area at an airport may be presented relative to the risk of exposure when dining indoor at a restaurant (outside of the airport). The value of the relative risk index may be a percentage or multiple of a value representing the risk of exposure at the benchmark location. For example, the value of the relative risk index may be two times (2x) less likely to be exposed to the health risk relative to indoor dining in a restaurant in the jurisdiction (outside of the airport). As such, the risk values for the nodes may be relative to estimated risks at one or more benchmark locations. Optionally, the risk assessment system 100 may permit a user to select one or more of the benchmark locations used to generate the relative risk index for a given node. For example, a user may select at least one benchmark location from a list stored in the memory 112. Once the benchmark location is selected, the control unit 102 may display a relative risk index that indicates the risk of exposure to the health threat at the node relative to the risk of exposure at the one or more selected benchmark locations. Optionally, the control unit 102 may display a risk value for the node next to a risk value associated with the benchmark location that is selected by the user, which enables the user to make a risk comparison. In an alternative example, the risk indices may be absolute risk indices that are not based on, or relative to, estimated risks at alternative locations.

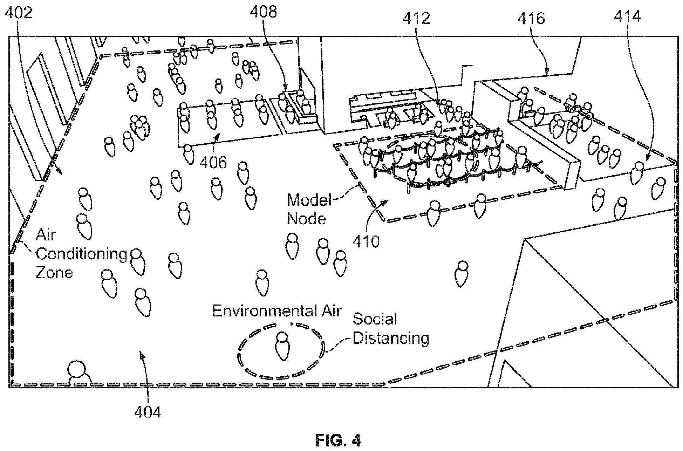

Figure 4 illustrates an air conditioning zone 402 within an airport according to an example. The background environmental air exposure risk sub-model 220 shown in Figure 2 may model the risk of a health threat from sharing a volume of air that is treated by a common air conditioning system. The air conditioning zone 402 may be shared by multiple passengers and staff in different nodes 304. For example, the air conditioning zone 402 encompasses a lobby node 404, a check-in queue node 406, a check-in counter node 408, a bag drop-off queue node 410, a counter node 412, a security queue node 414, and a checkpoint node 416. The background environmental air exposure risk sub-model 220 may accumulate contributions from people in all nodes serviced by the same air conditioning zone 402. The sub-model 220 may use a load factor to determine the number of people in each node. The contribution to the relative risk index from each node may be influenced by disease prevalence and activity level within the node. The activity level may be influenced by the set of control measures in place and the behavioral compliance with the control measures. For example, the relative risk index for a node can be reduced with control measures such as mask use, increased air change rate, reduced occupancy limits, and/or a reduction in the disease prevalence in the relevant jurisdiction.

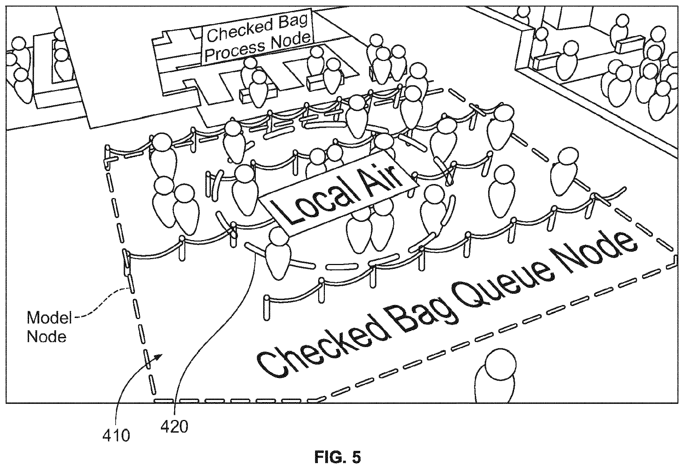

Figure 5 illustrates an enlarged view of the checked bag queue node 410 shown in Figure 4. The local air exposure risk sub-model 222 of Figure 2 may model the local risk of exposure attributed to proximity to other people in the same node. For example, the sub-model 222 may implement a risk index based on a circular area 420 (or cylindrical three-dimensional zone), which defines a "local air" region surrounding a passenger. The radius of the circle may be between 3 m and 5 m, for example. The passenger at the center of the local air region is potentially exposed to respiratory plumes from infected individuals nearby, such as individuals in the same node 410. The risk index may factor activity and occupancy within the area 420, such as air exchange rate, breathing level, air flow rate, and concentration of exhaled infectious quanta. In an example, the risk index may only consider contributions from people within the node 410. The sub-model 222 may use load factor, floor area, positioning (e.g., evenly spaced, clumped, queued), and/or the like to determine people density in the node and the number of people in the local area 420 or zone. The relative risk index for the node 410 may be influenced by disease prevalence and activity level within the node 410. The relative risk index for the node 410 can be reduced with control measures, as described above.

The surface borne exposure risk sub-model 224 shown in Figure 2 may consider the rate of touch of common surfaces and personal items, such as phones, to provide a relative risk index. The sub-model 224 may model the movement of pathogens between surfaces and hands, and then onto mucous membranes of a person. The sub-model 224 may incorporate fixed parameters, such as mucous membrane touch rates, die-off rates of pathogens on surfaces and hands, and/or the like. The sub-model 224 may determine a cumulative dosage across multiple nodes when aggregating the data from each individual node in the chain.

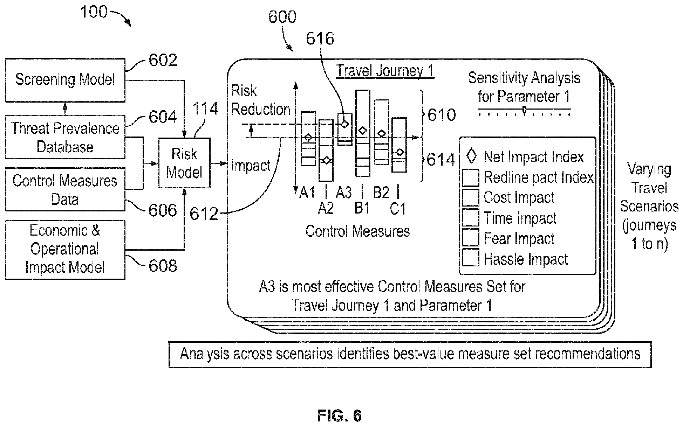

Figure 6 illustrates a portion of the risk assessment system 100 including graphical analytics 600 according to an example. The risk model 114 of the system 100 may be communicatively connected to other databases and models, such as a screening model 602, a threat prevalence database 604, a control measures database 606, and an economic and operational impact model 608. The threat prevalence database 604 may include the jurisdictional prevalence data 204 shown in Figure 2. The control measures database 606 may include a list of possible control measures and data about how each control measure in the list would affect the risk of exposure to the health threat. The economic and operational impact model 608 provides information (e.g., quantitative and/or qualitative) about the perceived impact that each control measure would have on the travel ecosystem if implemented. The impact model 608 is also shown in Figure 2. Potential impacts can include added costs, time delays, and demands, as well as emotional impacts, such as negative sentiments due to fear and hassle (e.g., additional measures that must be taken which reduce travel efficiency for a traveler and may require additional performance by the traveler). These impacts are listed in Figure 2. The model 114 may analyze other adverse impacts besides the ones listed.

In an example, the risk model 114 may incorporate the economic and operational impact model 608 by assigning a score or a ranking for each control measure along different impact categories. For example, the control measure of reduced occupancy limits may be assigned a score or ranking (among other control measures) with respect to economic cost to implement, potential delay to travelers and reduced throughput, perceived additional demand on parties to implement, level of fear, and level of hassle. Mask-wearing and the other control measures would similarly receive assigned scores or ranking for each category.

The analytics 600 shown in Figure 6 potentially could be displayed in the user interface 116 on the display device 104. However, the analytics 600 may show that different sets of control measures can be compared to recommend or select one set over other sets. For a single journey of a traveler (e.g., Journey 1) or stage of a journey, various different sets of control measures may be analyzed using the risk model 114. The analyzed sets may include a first set A1, a second set A2, a third set A3, a fourth set B1, a fifth set B2, and a sixth set C1. Each set differs from the other sets by at least one control measure and/or by the number of control measures in the set.

The processors 110 may use the risk model 114 to calculate the relative risk indices for the nodes in the chain for each set of control measures. The processors 110 may then aggregate the risk indices to determine the risk value for the stage (or the entire journey). The risk reduction values (e.g., bars) 610 in Figure 6 are above the horizontal line 612 and represent an increase in threat avoidance or risk reduction by implementing the specific set of control measures versus a baseline travel scenario in which no control measures are implemented. The risk reduction values 610 may represent risk values 610 associated with each respective set of control measures in place. For example, the risk model 114 may calculate the risk indices without control measures to use for the baseline case to which the experimental cases are compared. As described above, each set of control measures may be associated with adverse impacts depending on the number and type of control measures in the set. The impact bars 614 below the horizontal line 612 represent the adverse impact 614 that the specific set of control measures are expected to have on the travel ecosystem, such as on travelers, flight crew, airline workers, and others involved in the travel industry.

As shown in Figure 6, the set of control measures B1 is indicated by the risk model 114 to provide a greater risk reduction over the baseline than the other sets of control measures tested. However, B1 also has the second most adverse impact. To account for the adverse impact, the processors 110 may determine the net value for each set of control measures by essentially subtracting the adverse impact 614 from the risk reduction 610. Stated differently, the processors 110 determine which tested set of control measures provides the greatest risk reduction relative to the adverse impact. In this case, the set A3 is the most effective set of control measures because it shows a greater net value 616 (e.g., net impact index) than the other tested sets of control measures, even though it does not have the greatest risk reduction. In this example, the A3 set of control measures has the greatest net value 616, which indicates that the A3 set is preferred over the other sets of control measures in the particular conditions.

According to an example, the processors 110 may generate a control signal to recommend, via a visual display, audible message, or the like, implementing the A3 set of control measures. The recommendation may be made by highlighting graphics associated with the A3 set. Alternatively, the processors 110 may display analytics similar to the analytics 600 in Figure 6, to inform an observer that the net impact index for A3 is greater than the other tested sets, to enable the observer to quickly make the conclusion to use A3. Optionally, the control signal may control the communication device 108 to transmit a wireless message to a computer device of a user, for the computer device in receipt of the message to notify the user of the risk value.

In a non-limiting example, the risk model 114 may perform the following calculations to achieve the results described herein.

The number of people $n_i$ at node $i$ varies with the current operating load of the airport and is given by:

$$n_i = L\,c_i + s_i \qquad (1)$$

where $L$ represents the current operating load of the airport, and $0 < L \le 1$. The variable $c_i$ represents the passenger capacity of node $i$, meaning the maximum number of people in the node. The variable $s_i$ represents the typical number of airport/airline/concession staff or employees in node $i$. One assumption of the model may be that the value of $s_i$ remains fixed even as the airport operating level changes.

The density of people at node $i$ ($\sigma_{N,i}$), in people per m², is dependent on whether people are evenly spread, clumped or queued at the node and is given by:

$$\sigma_{N,i} = \begin{cases} \dfrac{n_i}{A_i}, & d_i = \text{even} \\[2mm] \sigma_C \dfrac{n_i}{A_i}, & d_i = \text{clumped} \\[2mm] \sigma_Q, & d_i = \text{queue} \end{cases} \qquad (2)$$

where $A_i$ is the floor area of node $i$, in m²; $\sigma_C$ is a constant giving the people density multiplier for clumping; and $\sigma_Q$ is a constant giving people density value for queues, in people per m². Clumping is the tendency for people to stand closer together in groups rather than evenly spread through an area. A typical value for $\sigma_C$ is 1.5, and a typical value for $\sigma_Q$ is 2. The variable $d_i$ represents the behavior of people in node $i$ with regard to people density, where $d_i \in \{\text{even, clumped, queue}\}$.

The number of people $S_i$ within a social distancing unit at node $i$ is then given by:

$$S_i = \pi r_S^2 \sigma_N, \qquad (3)$$

where $r_S$ is the social distance radius, in m, and may be 1.5 m, 1.8 m (6 ft), or 2 m.

The effect of social distancing could be modelled by simply setting $S_i = 1$. However, this is an oversimplification. As the number of people in the node increases, the floor area of a node determines the point at which social distancing starts to break down. The social-distancing-limiting node population at node $i$ can be defined as the number of social distancing circles within the area of the node:

$$n_{L,i} = \frac{A_i}{\pi r_S^2} \qquad (4)$$

Social distancing acts as a counter to people clumping and queuing behavior, as people will tend not to clump and queues will maintain social distance while there is room to do so. When social distancing is applied, the density of people at node $i$ becomes:

$$\sigma_{S,i} = \begin{cases} \dfrac{n_i}{A_i}, & d_i \neq \text{queue} \\[2mm] \dfrac{1}{\pi r_S^2}, & d_i = \text{queue}, n_i \leq n_{L,i} \\[2mm] \dfrac{n_i}{A_i}, & d_i = \text{queue}, n_i > n_{L,i} \end{cases} \qquad (5)$$

Social distancing compliance can be incorporated by blending between the standard people density from equation (2) and the social distancing density from equation (5):

$$\sigma_i = c_s \sigma_{S,i} + (1 - c_s) \sigma_{L,i} \qquad (6)$$

The number of people $n_S$, within a social distancing unit at node $i$ can then be given by:

$$n_{S,i} = \pi r_S^2 \sigma_i \tag{7}$$

The time $\tau_i$ spent at node $i$, in hours, is given by:

$$\tau_i = \begin{cases} t_i, & q_i = \text{false} \\ t_i^{\min} + 2L\left(t_i - t_i^{\min}\right), & q_i = \text{true}, L \le 0.5 \\ t_i + 2(L - 0.5)\left(t_i^{\max} - t_i\right), & q_i = \text{true}, L > 0.5 \end{cases} \tag{8}$$

where $q_i$ is a Boolean indicating that the time spent at node $i$ depends on the current operating load of the airport. The variable $t_i^{\min}$ is the minimum time spent by a passenger, in hours, at node $i$. For nodes where $q_i$ is true, this is the time spent at the node when $L=0$. The variable $t_i^{\max}$ is the maximum time spent by a passenger, in hours, at node $i$. For nodes where $q_i$ is true, this is the time spent at the node when $L=1$. The variable $t_i$ is the most likely time spent by a passenger, in hours, at node $i$. For nodes where $q_i$ is true, this is the time spent at the node when $L=0.5$.

Disease prevalence is used to represent the number of infected people at each node in the airport. As this is a relative risk model, the prevalence is used at each node to compare infection levels. The infection level at node $i$ can be defined as:

$$P_i = \begin{cases} \left(P_p L c_i + P_l s_i\right) / n_i, & \text{for public side nodes} \\ \left(P_s L c_i + P_l s_i\right) / n_i, & \text{for secure side nodes} \end{cases} \tag{9}$$

where $P_p$ is the average disease prevalence for passengers in the public side of the airport; $P_s$ is the average disease prevalence for passengers in the secure side of the airport; and $P_l$ is the average disease prevalence around the location of the airport, i.e. for airport staff. The variable $s_i$ is the typical number of airport/airline/concession staff in node $i$.

Mask wearing compliance is assumed to be uniform across the airport. The effect of mask usage and mask compliance at node $i$ is given by:

$$p_{m,i} = \begin{cases} 1 - c_m(1 - p_m), & e_i = \text{false} \\ 1, & e_i = \text{true} \end{cases} \tag{10}$$

where $c_m$ is the average value for mask wearing compliance in the airport; $p_m$ is the average value for disease penetration through masks worn in the airport; and $e_i$ is a Boolean indicating that people are eating and/or drinking at node $i$ and hence not wearing masks. The ranges may be $0 \le p_m \le 1$ and $0 \le c_m \le 1$. A value of 0 for $p_m$ indicates that there is no penetration of virus particles through the mask, and 1 indicates complete penetration (e.g., no mask).

The following equation represents a method to calculate the relative behavioral risk posed to a particular individual by attending a specific event in a well-mixed room:

$$I_R = \frac{N \tau C_q Q_{b,l}^2 p_m^2}{\lambda_a V} \tag{11}$$

where $C_{q,l}$ is the average concentration of exhaled infection quanta, per m$^3$, by an infectious individual with breathing level $l$ ($l \in B$), and $Q_{b,l}$ is the average breathing flow rate, in m$^3$/h, of a person with breathing level $l$ ($l \in B$). The variable $V_v$ is the volume of the environment $v$, in m$^3$, and the variable $\lambda_a$, is the air change rate per hour of the environment $v$.

This model can be extended to add infection level from (9) in the numerator, representing the linear relationship between infection level and relative risk.

In an airport the well-mixed room is a compound volume and contains one or more nodes each with different numbers of people, different activities, and potentially different levels of mask use, all sharing the same volume of air (i.e. within the same air conditioning zone). When evaluating the risk of a node $i$, the contributions from other nodes $j$ can be summed, where $v_j = v_i$. An environmental risk index for the node $i$ can be defined as:

$$I_{E,i} = \frac{\tau_i Q_{b,l_i} p_{m,i}}{\left(\lambda_{a,v_i} + \lambda_{f,v_i}\right)V} \sum_{\substack{j=1 \\ v_j=v_i}}^{n} N_j P_j C_{q,b_j} Q_{b,l_j} p_{m,j}$$

$$(12)$$

where $\lambda_{f,v}$ is the filtration rate of the environment $v$.

The environmental risk index provides the background relative risk for the node $i$, but this will be identical for any node $j$ where $vj=vi$. However, within the well-mixed room the local risk at any location will be proportional to the number of people in close proximity to a particular individual and the activities they are undertaking. Using the respiratory plume risk radius, a local risk index for the node $i$ can be defined as:

$$I_{L,i} = \alpha \frac{\pi r_p^2 \sigma_i \tau_i P_i C_{q,b_i} Q_{b,l_i}^2 p_{m,i}^2}{\lambda_{a,v_i}}$$

$$(13)$$

where $\alpha$ is a tuning constant which will be used in the model to adjust for relative differences in the magnitudes of $I_R$ and $I_L$. The environmental and local contributions from (12) and (13) can be combined to define a relative risk index for airborne transmission at node $i$ as:

$$I_{A,i} = w_L I_{L,i} +, (1 - w_L) I_{E,i}$$

$$(14)$$

where $w_L, 0 < w_L < 1$, and can be used to adjust the relative contributions of local and environmental risks.

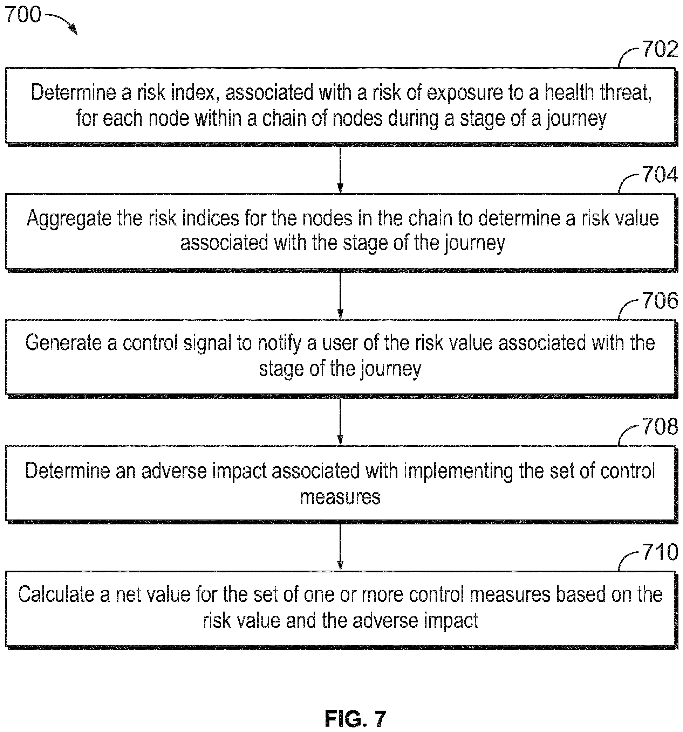

Figure 7 illustrates a flow chart 700 of a method for travel risk assessment according to an example of the present disclosure. Referring to Figures 1-6, the method begins at 702, at which a relative risk index associated with a risk of exposure to a health threat is determined for each node within a chain of nodes during a stage of a journey. The relative risk index is determined via a computer-implemented risk model. The relative risk index for each node is determined based on occupancy characteristics of the node, a set of one or more control measures in effect to reduce the risk of exposure in the node, and a health threat prevalence in a jurisdiction associated with the node.

At 704, the relative risk indices for the nodes in the chain are aggregated to determine a risk value associated with the stage of the journey. At 706, a control signal is generated to notify a user of the risk value associated with the stage of the journey.

At 708, an adverse impact associated with implementing the set of control measures is determined. At 710, a net value for the set of one or more control measures is calculated based on the risk value and the adverse impact. The net value may be displayed on a display device to notify the user of the net value. Optionally, the net value and/or the risk value associated with the specific set of control measures can be compared to other net values and/or risk values determined according to the method above except based on different sets of control measures. The method may include selecting the set of control measures which has a greater net value than the other sets of control measures as a recommended set of control measures. The method may include communicating the recommended set of control measures to at least one remote computing device, storing the recommended set of control measures in a record database, and/or generating a control signal to present the recommended set of control measures to a user via a display device, an audio device, or the like.

As described herein, certain examples of the present disclosure provide systems and methods that allow for identifying cost-effective and valuable control measures capable of reducing the risk of exposure to health threats when traveling while mitigating adverse impacts of implementing the control measures. The systems and methods may provide accurate modeling of travel conditions, improving the confidence at which control measures can be recommended over known health risk modeling efforts. Furthermore, the systems and methods may enhance traveler confidence in commercial aviation operations.

As used herein, the term "control unit," "central processing unit," "CPU," "computer," or the like may include any processor-based or microprocessor-based system including systems using microcontrollers, reduced instruction set computers (RISC), application specific integrated circuits (ASICs), logic circuits, and any other circuit or processor including hardware, software, or a combination thereof capable of executing the functions described herein. Such are exemplary only, and are thus not intended to limit in any way the definition and/or meaning of such terms. For example, the control unit 302 may be or include one or more processors that are configured to control operation, as described herein.

The control unit 102 is configured to execute a set of instructions that are stored in one or more data storage units or elements (such as one or more memories), in order to process data. For example, the control unit 102 may include or be coupled to one or more memories. The data storage units may also store data or other information as desired or needed. The data storage units may be in the form of an information source or a physical memory element within a processing machine.

The set of instructions may include various commands that instruct the control unit 102 as a processing machine to perform specific operations such as the methods and processes of the various examples of the subject matter described herein. The set of instructions may be in the form of a software program. The software may be in various forms such as system software or application software. Further, the software may be in the form of a collection of separate programs, a program subset within a larger program, or a portion of a program. The software may also include modular programming in the form of object-oriented programming. The processing of input data by the processing machine may be in response to user commands, or in response to results of previous processing, or in response to a request made by another processing machine.

The diagrams of examples herein may illustrate one or more control or processing units, such as the control unit 102. It is to be understood that the processing or control units may represent circuits, circuitry, or portions thereof that may be implemented as hardware with associated instructions (e.g., software stored on a tangible and non-transitory computer readable storage medium, such as a computer hard drive, ROM, RAM, or the like) that perform the operations described herein. The hardware may include state machine circuitry hardwired to perform the functions described herein. Optionally, the hardware may include electronic circuits that include and/or are connected to one or more logic-based devices, such as microprocessors, processors, controllers, or the like. Optionally, the control unit 102 may represent processing circuitry such as one or more of a field programmable gate array (FPGA), application specific integrated circuit (ASIC), microprocessor(s), and/or the like. The circuits in various examples may be configured to execute one or more algorithms to perform functions described herein. The one or more algorithms may include aspects of examples disclosed herein, whether or not expressly identified in a flowchart or a method.

As used herein, the terms "software" and "firmware" are interchangeable, and include any computer program stored in a data storage unit (for example, one or more memories) for execution by a computer, including RAM memory, ROM memory, EPROM memory, EEPROM memory, and non-volatile RAM (NVRAM) memory. The above data storage unit types are exemplary only, and are thus not limiting as to the types of memory usable for storage of a computer program.

Further, the disclosure comprises examples according to the following clauses:

Clause 1. A system comprising:

one or more processors configured to determine a risk index associated with a risk of exposure to a health threat for each node within a chain of nodes during a stage of a journey, wherein the risk index for each node is based on occupancy characteristics of the node, a set of one or more control measures in effect to reduce the risk of exposure in the node, and a health threat prevalence in a jurisdiction associated with the node;

wherein the one or more processors are further configured to aggregate the risk indices for the nodes to determine a risk value associated with the stage of the journey, and to generate a control signal to notify a user of the risk value associated with the stage of the journey.

Clause 2. The system of Clause 1, further comprising a user interface on a display device, wherein the one or more processors are configured to generate the control signal to display the risk value associated with the stage on the user interface.

Clause 3. The system of Clause 1 or Clause 2, wherein the stage of the journey is within an airport.

Clause 4. The system of Clause 3, wherein the one or more processors are further configured to determine a risk index associated with the risk of exposure to the health threat for each of multiple nodes within a chain of nodes during a second stage of the journey, the second stage representing one of transport to the airport, at least one flight segment departing from the airport, at least one flight segment arriving to the airport, or transport from the airport.

Clause 5. The system of Clause 4, wherein the one or more processors are further configured to aggregate the risk indices for the nodes during the second stage of the journey, and generate a control signal to notify the user of the risk value associated with the second stage of the journey.

Clause 6. The system of Clause 3, wherein the health threat prevalence in the jurisdiction associated with the node is a weighted sum of a prevalence value of a local jurisdiction that encompasses the airport and respective prevalence values of jurisdictions from which flight passengers arrive at the airport.

Clause 7. The system of any of Clauses 1-6, wherein the risk index for each node is also based on a behavior parameter indicative of a measure of behavioral compliance of people in the respective node to the set of one or more control measures in effect.

Clause 8. The system of any of Clauses 1-7, wherein the risk index for each node is also based on air flow characteristics of the node.

Clause 9. The system of any of Clauses 1-8 wherein the one or more processors are further configured to determine an adverse impact associated with implementing the set of control measures, and to calculate a net value for the set of control measures based on the risk value and the adverse impact.

Clause 10. The system of any of Clauses 1-9, wherein each node in the chain of nodes is within a common building or vehicle.

Clause 11. The system of any of Clauses 1-10, wherein the one or more processors are further configured to determine the chain of nodes encountered by the traveler by predicting movement and activity of the traveler during the stage of the journey.

Clause 12. The system of any of Clauses 1-11, wherein the risk index that is determined for each node is a relative risk index that represents a risk of exposure at the respective node compared to a risk of exposure at one or more benchmark locations outside of the stage of the journey.

Clause 13. The system of any of Clauses 1-12, wherein the set of one or more control measures in effect includes multiple different types of control measures in effect, such that the risk index for each node is based on the multiple different types of the control measures in effect.

Clause 14. A method comprising:

determining, via a computer-implemented model, a risk index associated with a risk of exposure to a health threat for each node within a chain of nodes during a stage of a journey, wherein the risk index for each node is determined based on occupancy characteristics of the node, a set of one or more control measures in effect to reduce the risk of exposure in the node, and a health threat prevalence in a jurisdiction associated with the node;

aggregating the risk indices for the nodes to determine a risk value associated with the stage of the journey; and

generating a control signal to notify a user of the risk value associated with the stage of the journey.

Clause 15. The method of Clause 14, further comprising displaying the risk value associated with the stage within a user interface on a display device.

Clause 16. The method of Clause 14 or Clause 15, wherein the stage of the journey is within an airport, and the method further comprises determining a risk index associated with the risk of exposure to the health threat for each of multiple nodes within a chain of nodes during a second stage of the journey, the second stage representing one of transport to the airport, at least one flight segment departing from the airport, at least one flight segment arriving to the airport, or transport from the airport.

Clause 17. The method of any of Clauses 14-16, wherein the risk index for each node is also determined based on a behavior parameter indicative of a measure of behavioral compliance of people in the respective node to the set of one or more control measures.

Clause 18. The method of any of Clauses 14-17, further comprising determining an adverse impact associated with implementing the set of control measures; and calculating a net value for the set of control measures based on the risk value and the adverse impact.

Clause 19. The method of Clause 18, further comprising comparing the net value for the set of control measures to other net values associated with the stage of the journey that are calculated based on different sets of control measures, and selecting the set of control measures that has a greater net value than the other sets of control measures as a recommended set of control measures.

Clause 20. The method of any of Clauses 14-19, further comprising determining the chain of nodes by predicting movement and activity of a traveler during the stage of the journey.

Clause 21. The method of any of Clauses 14-20, further comprising aggregating the risk indices for the nodes during the second stage of the journey, and generating a control signal to notify the user of the risk value associated with the second stage of the journey.

Clause 22. A system comprising:

a display device; and

one or more processors communicatively connected to the display device, the one or more processors configured to determine a risk index associated with a risk of exposure to a health threat for each node within a chain of nodes during a journey, wherein the risk index for each node is based on occupancy characteristics of the node, a set of one or more control measures in effect to reduce the risk of exposure in the node, and a health threat prevalence in a jurisdiction associated with the node;

wherein the one or more processors are further configured to aggregate the risk indices for the nodes to determine a risk value associated with the journey and the set of one or more control measures, and to determine an adverse impact associated with implementing the set of one or more control measures;

wherein the one or more processors are configured to calculate a net value for the set of control measures based on the risk value and the adverse impact, and display the net value on the display device.

While various spatial and directional terms, such as top, bottom, lower, mid, lateral, horizontal, vertical, front and the like can be used to describe examples of the present disclosure, it is understood that such terms are merely used with respect to the orientations shown in the drawings. The orientations can be inverted, rotated, or otherwise changed, such that an upper portion is a lower portion, and vice versa, horizontal becomes vertical, and the like.

As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein.

It is to be understood that the above description is intended to be illustrative, and not restrictive. For example, the above-described examples (and/or aspects thereof) can be used in combination with each other. In addition, many modifications can be made to adapt a particular situation or material to the teachings of the various examples of the disclosure without departing from their scope. While the dimensions and types of materials described herein are intended to define the parameters of the various examples of the disclosure, the examples are by no means limiting and are exemplary examples. Many other examples will be apparent to those of skill in the art upon reviewing the above description. The scope of the various examples of the disclosure should, therefore, be determined with reference to the appended claims, along with the full scope of equivalents to which such claims are entitled. In the appended claims and the detailed description herein, the terms "including" and "in which" are used as the plain-English equivalents of the respective terms "comprising" and "wherein." Moreover, the terms "first," "second," and "third," etc. are used merely as labels, and are not intended to impose numerical requirements on their objects.

This written description uses examples to disclose the various examples of the disclosure, including the best mode, and also to enable any person skilled in the art to practice the various examples of the disclosure, including making and using any devices or systems and performing any incorporated methods. The patentable scope of the various examples of the disclosure is defined by the claims, and can include other examples that occur to those skilled in the art. Such other examples are intended to be within the scope of the claims if the examples have structural elements that do not differ from the literal language of the claims, or if the examples include equivalent structural elements with insubstantial differences from the literal language of the claims.

**Claims**

1. A system (100) comprising:

   one or more processors (110) configured to determine a risk index (226) associated with a risk of exposure to a health threat for each node (304) within a chain (398) of nodes during a stage (202) of a journey, wherein the risk index for each node is based on occupancy characteristics of the node, a set of one or more control measures in effect to reduce the risk of exposure in the node, and a health threat prevalence in a jurisdiction associated with the node;
   wherein the one or more processors are further configured to aggregate the risk indices for the nodes to determine a risk value (610) associated with the stage (202) of the journey, and to generate a control signal to notify a user of the risk value associated with the stage of the journey.

2. The system (100) of claim 1, further comprising a user interface (116) on a display device (104), wherein the one or more processors (110) are configured to generate the control signal to display the risk value (610) associated with the stage (202) on the user interface.

3. The system (100) of claim 1 or 2, wherein the stage (202) of the journey is within an airport.

4. The system (100) of claim 3, wherein the one or more processors (110) are further configured to determine a risk index (226) associated with the risk of exposure to the health threat for each of multiple nodes (304) within a chain (398) of nodes during a second stage (202) of the journey, the second stage representing one of transport to the airport, at least one flight segment departing from the airport, at least one flight segment arriving to the airport, or transport from the airport.

5. The system (100) of claim 4, wherein the one or more processors (110) are further configured to aggregate the risk indices (226) for the nodes (304) during the second stage (202) of the journey, and generate a control signal to notify the user of the risk value (610) associated with the second stage of the journey.

6. The system (100) of claim 3, wherein the health threat prevalence in the jurisdiction associated with the node (304) is a weighted sum of a prevalence value (204) of a local jurisdiction that encompasses the airport and respective prevalence values of jurisdictions from which flight passengers arrive at the airport.

7. The system (100) of any of claims 1-6, wherein the risk index (226) for each node (304) is also based on a behavior parameter (219) indicative of a measure of behavioral compliance of people in the respective node to the set of one or more control measures in effect.

8. The system (100) of any of claims 1-7, wherein the risk index (226) for each node (304) is also based on air flow characteristics of the node.

9. The system (100) of any of claims 1-8, wherein the one or more processors (110) are further configured to determine an adverse impact (614) associated with implementing the set of control measures, and to calculate a net value (616) for the set of one or more control measures based on the risk value (610) and the adverse impact.

10. The system (100) of any of claims 1-9, wherein each node (304) in the chain (398) of nodes is within a common building or vehicle.

11. The system (100) of any of claims 1-10, wherein the one or more processors (110) are configured to determine the chain (398) of nodes by predicting movement and activity of a traveler during the stage (202) of the journey.

12. The system (100) of any of claims 1-11, wherein the risk index (226) that is determined for each node (304) is a relative risk index that represents a risk of exposure at the respective node compared to a risk of exposure at one or more benchmark locations outside of the stage (202) of the journey.

13. The system (100) of any of claims 1-12, wherein the set of one or more control measures in effect includes multiple different types of control measures in effect, such that the risk index (226) for each node (304) is based on the multiple different types of the control measures in effect.

14. A method comprising:

determining, via a computer-implemented model, a risk index associated with a risk of exposure to a health threat for each node within a chain of nodes during a stage of a journey, wherein the risk index for each node is determined based on occupancy characteristics of the node, a set of one or more control measures in effect to reduce the risk of exposure in the node, and a health threat prevalence in a jurisdiction associated with the node; aggregating the risk indices for the nodes to determine a risk value associated with the stage of the journey; and generating a control signal to notify a user of the risk value associated with the stage of the journey.

15. A system (100) comprising:

a display device (104); and one or more processors (110) communicatively connected to the display device, the one or more processors configured to determine a risk index (226) associated with a risk of exposure to a health threat for each node (304) within a chain (398) of nodes during a journey, wherein the risk index for each node is based on occupancy characteristics of the node, a set of one or more control measures in effect to reduce the risk of exposure in the node, and a health threat prevalence in a jurisdiction associated with the node; wherein the one or more processors are further configured to aggregate the risk indices for the nodes to determine a risk value (610) associated with the journey and the set of one or more control measures, and to determine an adverse impact (614) associated with implementing the set of one or more control measures; wherein the one or more processors are configured to calculate a net value (616) for the set of control measures based on the risk value and the adverse impact, and display the net value on the display device.

100

102

104

**Control unit**

110 — Processor(s)

112 — Memory

114 — Risk Model

**Display Device**

User Interface

116

Input

106

Communication Device

108

**FIG. 1**

114 ~

Deterministic Compartmental Model Elements ———————— (A)

202

**Journey Phase Node Clusters**

204

| 202a | Transport to Airport |
| 202b | Departure Airport |
| 202c | Aircraft Flight Segments |
| 202d | Transit/Transfer Airport |
| 202e | Arrival Airport |
| 202f | Transport from Airport |

**Disease Prevalence**

| Prevalence Rating | Daily Incidence per 100,000 population |
|---|---|
| very low | <0.1 |
| low | 0.1-1 |
| medium | 1-10 |
| high | 10-25 |
| very high | >25 |

206 ~

**Prevalence Mixing**

Local Country Prevalence

| Departing Passengers | Flight Crew | Airport Workers |
|---|---|---|

Origin Country Prevalence weighted by % traffic

| Arriving Passengers |
|---|

210         214         216                 208

**FIG. 2**

Ⓐ ——————————————————————————————————————————————↓ 218 ↓

High Level Parameters of Risk

| Activity Level | Surface Touches | Viral Prevalence |

219 — | Behaviors | Air Quality | Area Layout |

| People Density | Operating Load | Time in Area |

——222

220 — | Background Air Exposure Risk | Local Air Exposure Risk | Surface Exposure Risk | ——224

Risk Index ——226

| Airborne Exposure Reduction | Surface Exposure Reduction | Viral Prevalence Reduction |

——608

Impact

| Cost | Fear | Demand | Time | Hassle |

**FIG. 2**
**CONTINUED**

20

FIG. 3

**FIG. 3**
**CONTINUED**

**FIG. 4**

Checked Bag
Process Node

Local Air

Checked Bag Queue Node

Model
Node

410    420

**FIG. 5**

FIG. 6

700 ⟍

┌─────────────────────────────────────────────────────────────────────────┐ ⌐702
│ Determine a risk index, associated with a risk of exposure to a health threat, │
│ for each node within a chain of nodes during a stage of a journey │
└─────────────────────────────────────────────────────────────────────────┘

│

┌─────────────────────────────────────────────────────────────────────────┐ ⌐704
│ Aggregate the risk indices for the nodes in the chain to determine a risk value │
│ associated with the stage of the journey │
└─────────────────────────────────────────────────────────────────────────┘

│

┌─────────────────────────────────────────────────────────────────────────┐ ⌐706
│ Generate a control signal to notify a user of the risk value associated with the │
│ stage of the journey │
└─────────────────────────────────────────────────────────────────────────┘

│

┌─────────────────────────────────────────────────────────────────────────┐ ⌐708
│ Determine an adverse impact associated with implementing the set of control │
│ measures │
└─────────────────────────────────────────────────────────────────────────┘

│

┌─────────────────────────────────────────────────────────────────────────┐ ⌐710
│ Calculate a net value for the set of one or more control measures based on the │
│ risk value and the adverse impact │
└─────────────────────────────────────────────────────────────────────────┘

**FIG. 7**

Europäisches
Patentamt

European
Patent Office

Office européen
des brevets

# EUROPEAN SEARCH REPORT

Application Number

EP 22 16 6727

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHUOJIE HUANG ET AL: "Web-based GIS: the vector-borne disease airline importation risk (VBD-AIR) tool", INTERNATIONAL JOURNAL OF HEALTH GEOGRAPHICS, BIOMED CENTRAL LTD, LONDON, GB, vol. 11, no. 1, 14 August 2012 (2012-08-14), page 33, XP021121533, ISSN: 1476-072X, DOI: 10.1186/1476-072X-11-33 * page 1, paragraph Abstract – page 3, paragraph Data * * page 6, paragraph Climate and other datatsets – page 7, paragraph Risk assessments * * figure 4 * | 1-15 | INV. H04L67/12 H04W4/029 H04W4/90 G16H50/80 |
| A | ALSHAMMARI SULTANAH M ET AL: "Disease Spread Simulation To Assess The Risk Of Epidemics During The Global Mass Gathering Of Hajj Pilgrimage", 2019 WINTER SIMULATION CONFERENCE (WSC), IEEE, 8 December 2019 (2019-12-08), pages 215-226, XP033718047, DOI: 10.1109/WSC40007.2019.9004669 [retrieved on 2020-02-19] * paragraphs [0002] – [02.5]; figure 1 * * paragraph [03.2] * | 1-15 | TECHNICAL FIELDS SEARCHED (IPC) H04L H04W G16H |
| A | WO 2021/016391 A1 (BROAD INST INC [US]; HARVARD COLLEGE [US]) 28 January 2021 (2021-01-28) * paragraphs [0002] – [0006] * * paragraphs [0038] – [0053]; figure 1 * | 1-15 | |

The present search report has been drawn up for all claims

| Place of search | Date of completion of the search | Examiner |
|---|---|---|
| Munich | 4 August 2022 | Günther, Steffen |

EPO FORM 1503 03.82 (P04C01)

## ANNEX TO THE EUROPEAN SEARCH REPORT
## ON EUROPEAN PATENT APPLICATION NO.

EP 22 16 6727

This annex lists the patent family members relating to the patent documents cited in the above-mentioned European search report.
The members are as contained in the European Patent Office EDP file on
The European Patent Office is in no way liable for these particulars which are merely given for the purpose of information.

04-08-2022

| Patent document cited in search report | Publication date | Patent family member(s) | | Publication date |
|---|---|---|---|---|
| WO 2021016391 A1 | 28-01-2021 | EP | 4004863 A1 | 01-06-2022 |
| | | US | 2021050116 A1 | 18-02-2021 |
| | | US | 2021151198 A1 | 20-05-2021 |
| | | WO | 2021016391 A1 | 28-01-2021 |

EPO FORM P0459

For more details about this annex : see Official Journal of the European Patent Office, No. 12/82